(19) Europäisches Patentamt
European Patent Office
Office européen des brevets

(11) Publication number: **0 424 851 A1**

(12) **EUROPEAN PATENT APPLICATION**

(21) Application number: **90120241.6**

(22) Date of filing: **22.10.90**

(51) Int. Cl.5: **C07D 519/00**, A61K 31/55, A61K 31/535, //(C07D519/00, 498:00,471:00),(C07D519/00, 498:00,487:00)

(30) Priority: **23.10.89 KR 8915203**

(43) Date of publication of application: **02.05.91 Bulletin 91/18**

(84) Designated Contracting States: **AT BE CH DE DK ES FR GB GR IT LI LU NL SE**

(71) Applicant: **KOREA RESEARCH INSTITUTE OF CHEMICAL TECHNOLOGY**
**100, Jang-Dong, Yuseong-Ku**
**Daejeon, 305-606(KR)**

(72) Inventor: **Kim, Wan Joo**
**383-7 Dorhyong-dong**
**Yuseong-Ku, Daejeon 305-606(KR)**
Inventor: **Park, Myung Hwan**
**431 Dorhyong-dong**
**Yuseong-Ku, Daejeon 305-606(KR)**
Inventor: **Oh, Jong Hoon**
**59 Gajang-dong**
**Seo-Ku, Daejeon 302-181(KR)**
Inventor: **Jung, Myung Hee**
**391 Dorhyong-dong**
**Yuseong-Ku, Daejeon 305-606(KR)**
Inventor: **Kim, Bong Jin**
**81.6 Byun-dong**
**Seo-Ku, Daejeon 301-190(KR)**

(74) Representative: **Becker, Heinrich Karl Engelbert, Dr. et al**
**HOECHST AKTIENGESELLSCHAFT Central Patent Department P.O. Box 80 03 20**
**W-6230 Frankfurt am Main 80(DE)**

(54) **Quinolone compounds and a process for their preparation.**

(57) Quinolone compounds of the general formula

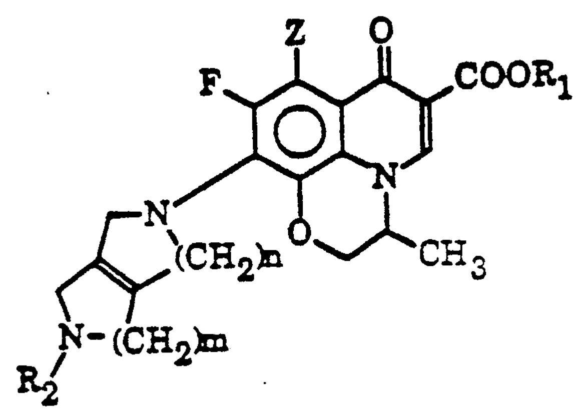

pharmaceutical compositions active against bacterial infections containing such compounds, processes for the manufacture of the quinolone compounds and the compositions and the use of the quinolone compounds for the manufacture of pharmaceutical compositions for the treatment of bacterial infections.

EP 0 424 851 A1

## QUINOLONE COMPOUNDS AND A PROCESS FOR THEIR PREPARATION

The present invention relates to new quinolone compounds of the following general formula I

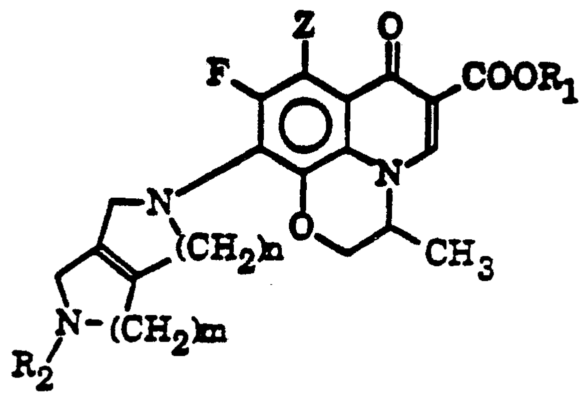

(I)

in which
Z represents hydrogen, halogen or amino,
$R_1$ represents hydrogen or a pharmaceutically acceptable cation,
$R_2$ represents hydrogen, lower alkyl or formyl,
m is an integer of 1 to 3, and
n is 1 or 2,
and, in case $R_1$ is hydrogen, pharmaceutically acceptable acid addition salts and the hydrates thereof.

These compounds have an excellent antibacterial activity and a broad antibacterial spectrum. The invention relates further to a process for their preparation.

If in the above formula I
Z is halogen, it may be e.g. chlorine or fluorine, preferably fluorine
$R_2$ is lower alkyl, it is preferably an alkyl group with 1 to 4 carbon atoms, especially methyl,
$R_1$ is a pharmaceutically acceptable cation, it may be an alkali metal cation, preferably sodium or potassium, an alkaline earth metal cation, preferably calcium or magnesium and further ammonium or the cation is an organic base, such as e.g. tri- or tetra-$C_1$-$C_4$-alkylammonium,
m is the integer of 1 to 3, it is preferably 1 and
n is an integer of 1 to 2, it is preferably 1.

Especially preferred compounds of the formula I are those, in which Z is hydrogen, $R_1$ is hydrogen, $R_2$ is hydrogen or $C_1$-$C_4$-alkyl, preferably methyl and m and n each are the integer 1.

Since the first introduction of nalidixic acid, quinolone carboxylic acid antibacterial agents have been effectively used to cure urinary tract infections due to their antibacterial activity against many aerobic Gram negative bacteria. Among these quinolone antibacterial agents, especially norfloxacin, ciprofloxacin, and ofloxacin are being widely used in hospitals nowadays in a variety of indications.

However, whereas these existing quinolone antibacterial agents have a high antibacterial activity against Gram negative bacteria, these agents have a disadvantage due to their lower antibacterial activity against Gram positive bacteria, such as Staphylococcus spp. or Streptococcus spp.

As result of the effort to solve the problems of the existing quinolone antibiotics as stated above, the present invention was made.

It is an object of the present invention to provide the new quinolone compounds of the formula I having a much better and wider range of antibacterial activity than the existing quinolone antibacterial agents against Gram positive and Gram negative bacteria and having an excellent antibacterial activity against clinically important methicillin-resistant bacteria.

It is also an object of the present invention to provide a process for the preparation of the new quinolone compounds of the formula I and the pharmaceutically acceptable salts thereof.

The process for the preparation of the compounds of the general formula I comprises

($a_1$) the step of condensing a compound of the general formula II

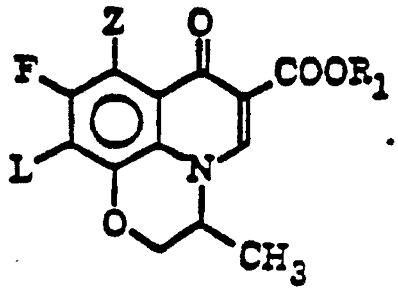

. (II)

wherein Z and $R_1$ are as defined above and L represents a leaving group with a diazabicycloamine of the general formula III

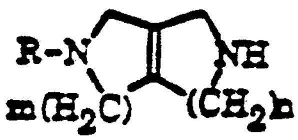

(III)

or its acid salt,
wherein m and n are as defined above and
R represents hydrogen, lower alkyl or a protecting group, or
($a_2$) the step of condensing a compound of the formula IIa

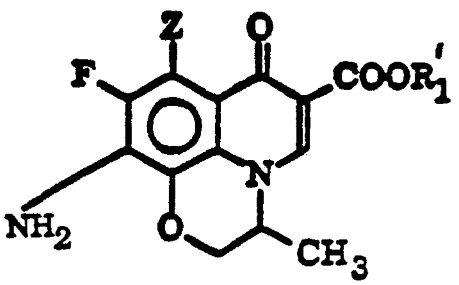

(IIa)

wherein Z has the meaning given above and $R'_1$ stands for a protecting group, with a compound of the formula IIIa

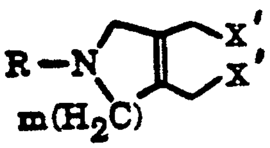

(IIIa)

wherein R and m are as defined above and X′ is a leaving group, whereby a compound of the general formula IV

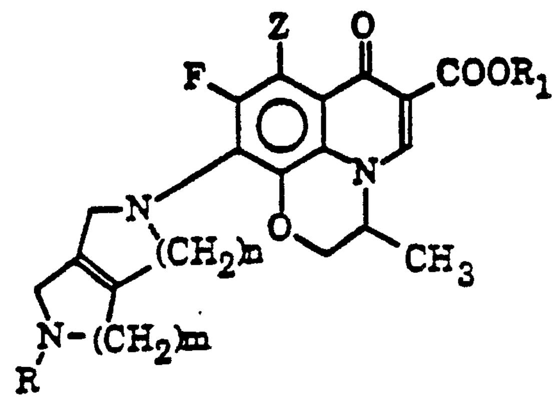

(IV)

is obtained,

in which Z, R, $R_1$, m and n are as defined above, but n is 1 for step $a_2$,

(b) removing the protecting groups and

(c) optionally replacing R = hydrogen by lower alkyl or formyl.

The leaving group L may be e.g. halogen, such as fluorine, chlorine or bromine, preferably fluorine, $C_1$-$C_4$-alkylsulfonyl, preferably ethylsulfonyl, or $C_1$-$C_4$-alkylsulfonyloxy.

As examples for the leaving group X′ may be mentioned halogen, such as chlorine, bromine or iodine, preferably bromine.

If R stands for a protecting group, it may in principle be each N-protecting group known from literature, e.g. from peptide or $\beta$-lactam chemistry, which can easily be split off in conventional manner, i.e. by solvolysis, including hydrolysis, hydrogenolysis or by reduction after the inventive condensation.

As examples for protecting groups removable by solvolysis may be mentioned arylsulfonyl, such as p-toluenesulfonyl or phenylsulfonyl; or alkoxycarbonyl such as ethoxy-, t-butoxy- or benzyloxycarbonyl.

The removal may be carried out in well-known manner in an appropriate solvent in the presence of an acid, such as e.g. hydrochloric, hydrobromic, sulfuric, acetic, trifluoroacetic or formic acid, or in the presence of a base, such as e.g. sodium or potassium hydroxide, sodium or potassium carbonate or sodium acetate. The solvent may be water, or - if necessary - an organic solvent, as e.g. ethanol, dioxane or acetic acid, alone or in mixture with water.

Examples for protecting groups removable by hydrogenolysis are benzyl or substituted benzyl; or arylsulfonyl, such as p-toluenesulfonyl or phenylsulfonyl.

These groups can also be split off in customary manner known from literature under different conditions, e.g. in a hydrogen stream in an inert solvent in the presence of a catalyst, as e.g. platinum, palladium or Raney nickel; or with e.g. zink in acetic acid or methanol.

It is also possible to remove protecting groups such as e.g. toluenesulfonyl or phenylsulfonyl by reduction, as for instance by $NaAlH_2(OCH_2CH_2OCH_3)_2$.

Because compounds of the formula III wherein R is a protecting group are preferably obtained by a cyclization reaction which can be generalized as follows

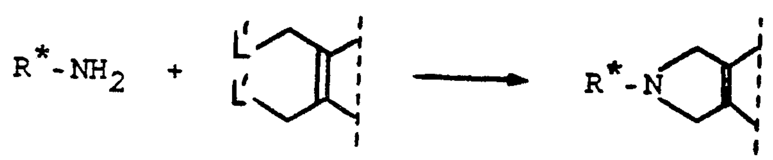

($R^*$ = protecting group, L' = leaving group)

protecting groups which can be introduced together with the nitrogen atom are therefore preferred, such as for instance arylsulfonyl, such as p-toluenesulfonyl or alkylsulfonyl, such as methanesulfonyl (both introduced as the corresponding sulfonamide), alkoxycarbonyl (introduced as the corresponding urethane) or acetyl (introduced as acetamide), preferably the p-toluenesulfonyl group.

A protecting group $R'_1$ may be any easily removable carboxylic acid protecting group known from literature, preferably a $C_1$-$C_4$-alkyl group, especially ethyl, which can be removed in known manner e.g.

under acidic or basic conditions.

As salts of the compound III salts with inorganic or organic acids can be used, such as e.g. hydrochloric acid, hydrobromic acid, formic acid or acetic acid, preferably hydrobromic acid.

The above reaction $a_1$ according to the present invention can be carried out advantageously in inert solvents such as e.g. acetonitrile, tetrahydrofuran, a lower alcohol, such as e.g. ethanol, chloroform, dimethylsulfoxide, dimethylformamide, pyridine, picoline or water, preferably acetonitrile or pyridine.

In order to neutralize the acid produced during the reaction, the free amine of the general formula III can be used in excess. Alternatively, a carbonate or bicarbonate of an alkaline metal or alkaline earth metal, for example the sodium or potassium carbonate or bicarbonate, an organic base, as for example triethylamine, diisopropylethylamine, pyridine, picoline or especially DBU (1,8-diazabicyclo[5.4.0]undec-7-ene) can be used alone or as a mixture.

The above condensation is preferably carried out in a broad temperature interval, as for example between room temperature and 150 °C, e.g. at reflux temperature of the used solvent, for about 1 to 10 hours.

If the variant $a_2$ is used, the reaction is also carried out in an invert solvent, such as e.g. dimethylformamide or acetonitrile, for example at room temperature for about 1 to 24 hours in the presence of a base, e.g. of anhydrous potassium carbonate.

The substituent $R_2$ in the compounds of the formula I can be introduced before or after the preparation of the compounds of formula IV. For example, monoalkylamines of the general formula III can be introduced by the above described processes and experimental conditions. Alternatively, by using methods known per se, for example the alkyl group can be introduced into compounds of the formula IV, wherein R is hydrogen, by the reaction with an alkyl halide, for example methyl or ethyl iodide, in an inert solvent, e.g. in dimethylformamide, in the presence of a base, e.g. of potassium carbonate. A methyl group can e.g. be introduced by reaction with formaline, preferably about 36 - 37 % aqueous formaline, and formic acid, e.g. under reflux for about 2 to 6 hours. A formyl group can be introduced by the reaction with formic acid in the presence of acetic anhydride.

When R is a protecting group such as e.g. benzyl or toluenesulfonyl, $R_2$ can only be introduced after removing this protecting group.

As starting materials of the present invention, the compounds of the general formula II are well known in the field of quinolone antibacterial agents and can be prepared according to the following references: L.A. Mitscher et al., J. Med. Chem., 30 2283 (1987).

The manufacture of starting materials of the type IIIa is described in the Preparations.

The diazabicycloamine compounds of general formula III, as 3,7-diazabicyclo[3.3.0]oct-1(5)-ene or 3,8-diazabicyclo[4.3.0]non-1(6)-ene or acid salts thereof, which are to be introduced onto the C-10 position of the quinolone compounds of formula II, are new compounds.

These compounds can for instance be prepared according to the following methods:

**Preparation of 3,7-diazabicyclo[3.3.0]oct-1(5)-ene**

**Method A**

The well known tetrakis(bromomethyl)ethylene [Reference:(1) A.C. Cope et al., J. Am. Chem. Soc., 80 , 5499 (1958), (2) P.W. Le Quesne et al., J. Org. Chem., 40 , 142 (1975)], is heated in a sealed tube with liquid ammonia and alcohol solvent. The above compound is obtained as the free amine.

**Method B**

Tetrakis(bromomethyl)ethylene is cyclized with p-toluene sulfonamide (or methanesulfonamie or acetmide) in the presence of a base and a polar solvent and then heated in the presence of a strong acid to remove the p-toluene sulfonyl group. The acid salt of the above compound is obtained.

**Preparation of 3,8-diazabicyclo[4.3.0]non-1(6)-ene**

3,4-Bis-bromomethyl-3-pyrroline is prepared by cyclization of tetrakis(bromomethyl)ethylene with one

equivalent of p-toluenesulfonamide. One of the two bromines is substituted by a cyanide group which is then reduced to the aminoethyl group. By cyclizing this compound in the presence of a base, nitrogen protected 3,8-diazabicyclo[4.3.0]non-1(6)-ene derivative is prepared.

Thereafter, the protecting group of this compound is removed in the presence of acid. Alternatively, the second protecting group, for example the benzyl group is introduced into the secondary amine of this compound, the first protecting group is selectively removed in the presence of an acid and then the second protecting group is removed by hydrogenolysis under acidic conditions. Thus, the acid salt of the above compound is obtained. When it is treated with alkali, the free amine is obtained.

In the process of the present invention, each protecting group can - as already mentioned above - be removed by conventional methods, e.g. by using an acid, as e.g. hydrobromic acid, or alkali such as e.g. sodium hydroxide or potassium hydroxide, or by hydrogenolysis. Thus, N-protecting group, such as toluenesulfonyl may preferably be removed by hydrobromic acid, for example 40 % hydrobromic acid, in the presence of phenol under reflux conditions for several hours.

The hydrogenolysis to remove e.g. a benzyl group can also be carried out in usual manner, for example by using 10 % Pd charcoal/hydrogen.

The present invention involves also the pharmaceutically acceptable acid addition salts and basic salts of the compounds of the general formula I. The basic salts can be formed with an alkaline metal, as e.g. sodium or potassium, ammonium, an alkaline earth metal, as e.g. calcium or magnesium, or organic amines, as e.g. tri- or tetraalkyl ammonium. These salts may be obtained e.g. by treatment with the corresponding base, e.g. sodium or potassium hydroxide or with metal salts, e.g. with sodium or potassium carbonate.

The acid addition salts can be formed with appropriate organic acids or inorganic acids. Suitable acids for salt formation are, for example, hydrochloric acid, sulfuric acid, phosphoric acid, acetic acid, citric acid, lactic acid, boric acid, malonic acid, salicyclic acid, malic acid, maleic acid, gluconic acid, fumaric acid, succinic acid, ascorbic acid and methanesulfonic acid, preferably hydrochloric acid.

The above salts can be prepared in conventional manner by treatment of the above free base form of the compounds of the general formula I with an excess amount of acid which can form mono- or di-salts. Thus, the hydrochloride may, for example, be obtained by the treatment with 20 % hydrochloric acid in isopropanol, or with isopropanol saturated with HCl-gas, or with concentrated hydrochloric acid in methanol or ethanol, the formic acid salt e.g. by treamtnet with formic acid.

The compounds of the formula I may exist in their anhydrous form or as hydrates. Hydrates can be obtained during the isolation or by conventional methods.

The present invention involves also antibacterial compositions for oral or parenteral administration to human beings and animals, containing an effective amount of one or more of the quinolone compounds represented by the general formula I or salts thereof as active components, together with the usual pharmacologically acceptable excipients or diluents.

Pharmaceutical compositions which contain one or more compounds of the general formula I as the active compound can be prepared by mixing the compound(s) of the general formula I with one or more pharmacologically acceptable excipients or diluents, such as, for example, fillers, emulsifiers, lubricants, flavor-correcting agents, dyestuffs or buffer substances, and converting the mixture into a suitable galenical formulation form, such as, for example, tablets, dragees, capsules, granules, pellets, syrups, suspensions or a solution or suspension suitable for parenteral administration. Examples of commonly used excipients or diluents which may be mentioned are tragacanth, lactose, talc, starch, agar-agar, polyglycols, ethanol and water. Suspensions or solutions in water can preferably be used for parenteral administration. It is also possible to administer the active compounds as such, without excipients or diluents, in a suitable form, for example in capsules.

Suitable doses of the compounds of the general formula I are about 0.1 to 1.5 g/day, preferably 0.2 to 0.8 g/day, for an adult having a body weight of about 60 kg. The dose may vary depending e.g. upon the body weight, age or symptoms of the patient. Thus, individual doses or, in general, multiple doses may be administered, it being possible for the individual dose to contain the active compound in an amount of about 100 to 750 mg.

The products according to the invention can also be used in combination with other active compounds, for example from the series of penicillins, aminoglycosides, cephalosporins or other compounds which influence bacterial infections, such as, for example, antipyretic agents, analgesic agents or antiphlogistic agents.

The following examples and preparations illustrate the present invention without limiting the scope of the invention.

Preparations

Preparation 1 : Preparation of 3,7-bis-ρ-toluenesulfonyl-3,7-diazabicyclo[3.3.0]oct-1(5)-ene

30g of tetrakis (bromomethyl)ethylene and 30g of ρ-toluene sulfonamide were dissolved in 400 ml of dimethylformamide. 150g of potassium carbonate (or 50% sodium hydride 17g) was added and then stirred at room temperature for 24 hours. Thereafter, this reaction mixture was distilled under vacuum to remove solvents. By adding 30 ml of water and 100 ml of ethylacetate, 17g of the title compound was obtained as pale yellow powder (yield 50%).
Melting point : 250 °C (dec.)
$^1$H-NMR (DMSO-$d_6$, δ ppm) : 7.65 (4H, d, J = 8.08 Hz), 7.39 (4H, d, J = 8.08 Hz), 3.94 (8H, s), 2.40 (6H, s).
EIMS : m/z 418 ($M^+$, 1.3%), m/z 419 ($M^+$ + 1, 1.2%).

Preparation 2 : Preparation of 3,7-diazabicyclo[3.3.0]oct-1(5)-ene dihydrobromide

60 ml of 48% hydrobromic acid and 7g of phenol were added to 10.8g of 3,7-bis-ρ-toluenesulfonyl-3,7-diazabicyclo[3.3.0]oct-1(5)-ene, prepared in Preparation 1. The mixture was refluxed for 4 hours and cooled to room temperature. The aqueous phase was separated by adding 100 ml of chloroform and 50 ml of water. The aqueous phase was washed with chloroform (100 ml x 4) and decolorized with active carbon. The aqueous phase was concentrated under vacuum and the remained solid was washed with 1 : 1 methanol-ethyl ether solvent. 5g of the title compound was obtained as white solid (yield 71%).
Melting point : 220 °C (dec.)
$^1$H-NMR (DMSO-$D_2O$, δ ppm) : 4.06 (8H, s).
MS : m/z 110 ($M^+$).

Preparation 3 : Preparation of 3,7-diazabicyclo[3.3.0]oct-1(5)-ene

2.72g of 3,7-diazabicyclo[3.3.0]oct-1(5)-ene dihydrobromide, prepared in Preparation 2, was added to 10 ml of 10% aqueous sodium hydroxide solution. The mixture was concentrated under reduced pressure to remove water, and then extracted with ethyl ether several times and concentrated. 1g of the title compound was obtained (yield 90%).
$^1$H-NMR ($D_2O$, δ ppm) : 4.02 (8H, s).
MS : m/z 110 ($M^+$).

Preparation 4 : Preparation of 3,7-diazabicyclo[3.3.0]oct-1(5)-ene

0.7g of tetrakis (bromomethyl)ethylene was dissolved in 10 ml of methanol and 4 ml of liquid ammonia and sealed and heated in 180 °C oil bath for 8 hours. After cooling the reaction mixture to room temperature, ammonia was evaporated. The mixture was concentrated to remove methanol. 10 ml of absolute ethanol was added and the undissolved compound was filitered off to remove insoluble material. Ethanol was removed by vacuum distillation. 3 ml of 30% aqueous potassium hydroxide solution was added to the oil residue. The solution was extracted with tetrahydrofuran (THF, 5 ml x 3) and the obtained extrates were combined and dried ($Na_2SO_4$), concentrated to give 60mg of the title compound (yield 31%).
$^1$H-NMR (DMSO-$d_6$, δ ppm) : 4.04 (8H, s).
MS : m/z 110 ($M^+$).

Preparation 5 : Preparation of N-(ρ-toluenesulfonyl)-3,4-bis(bromomethyl)-3-pyrroline

19g of tetrakis (bromomethyl)ethylene and 9g of ρ-toluene sulfonamide were dissolved in 220 ml of dimethylformamide. 30g of anhydrous potassium carbonate was added and then stirred at room temperature for 20 hours. Thereafter solvent was removed by vacuum distillation. 50 ml of ethylacetate was added to obtain solid product, and solid product was purified by silica gel column chromatography. 12g of the title

compound was obtained (yield 60%).
Melting point : 170 ° C
$^1$H-NMR ($CDCl_3$, δ ppm) : 7.69 (2H, d, J = 8.2 Hz), 7.33 (2H, d, J = 8.2 Hz), 4.00 (4H, s), 3.15 (4H, s), 2.44 (s, 3H).

Preparation 6 : Preparation of N-(ρ-toluenesulfonyl)-3-(bromomethyl)-4-(cyanomethyl)-3-pyrroline

10g of N-(ρ-toluenesulfonyl)-3,4-bis(bromomethyl)-3-pyrroline, prepared in Preparation 5, was dissolved in 10 ml of dimethylsulfoxide (DMSO) and then heated in oil bath for 2 hours with refluxing. During the heating and refluxing, 1.5g of sodium cyanide was added by small portion. The reaction mixture was cooled to room temperature and poured into ice-water and then extracted with methylene chloride (200 ml x 3). The extracts were combined, dried over $Na_2SO_4$ and concentrated. The residue was purified by silica gel column chromatography. 5g of the title compound was obtained (yield 57%).
Melting point : 182 ° C
$^1$H-NMR ($CDCl_3$, δ ppm) : 7.71 (2H, d, J = 8.2 Hz), 7.36 (2H, d, J = 8.2 Hz), 4.01 (4H, s), 3.20 (2H, s), 3.06 (2H, s), 2.45 (3H, s).

Preparation 7 : Preparation of N-(ρ-toluenesulfonyl)-3-(aminoethyl)-4-(bromomethyl)-3-pyrroline

4g of N-(ρ-toluenesulfonyl)-3-(bromomethyl)-4-(cyanomethyl)-3-pyrroline, prepared in Preparation 6, was dissolved in 100 ml of ethyl ether. The solution was slowly added to the suspension of 1g of lithium aluminum hydride (LAH) in 20 ml of ethyl ether, and heated with refluxing for 3 hours. The reaction mixture was cooled by ice water. After adding 3 ml of water, it was stirred for 30 minutes and filtered off. The filtrate was concentrated. 2g of the title compound was obtained (yield 49%).
Melting point : 185 ° C
$^1$H-NMR ($CDCl_3$, δ ppm) : 7.84 (2H, d, J = 8.2 Hz), 7.46 (2H, d, J = 8.2 Hz), 4.20 (2H, g, J = 7 Hz), 4.06 (4H, s), 2.45 (3H, s), 2.26 (2H, t, J = 7 Hz).

Preparation 8 : Preparation of $N^8$-(ρ-toluenesulfonyl)-3,8-diazabicyclo[4.3.0]non-1(6)-ene

3.6g of $N^1$-(ρ-toluenesulfonyl)-3-(aminoethyl)-4-(bromomethyl-3-pyrroline, prepared in Preparation 7, was dissolved in 30 ml of dimethylformamide. 5g of anhydrous potassium carbonate was added to the solution and then stirred at room temperature for 18 hours. After concentrating the reaction mixture under reduced pressure to remove solvent, the mixture was extracted with methylene chloride (50 ml x 3). After mixing the obtained extracts, it was washed with water and concentrated. 2.5g of the title compound was obtained (yield 88%).
Melting point : 201 ° C
$^1$H-NMR ($CDCl_3$, δ ppm) : 7.80 (2H, d, J = 8.2 Hz), 7.44 (2H, d, J = 8.2 Hz), 4.05 (4H, s), 3.41 (2H, s), 2.92 (2H, t, J = 5.8 HZ), 2.44 (2H, t, J = 5.8 Hz).

Preparation 9 : Preparation of $N^3$-(benzyl)-$N^8$-(ρ-toluenesulfonyl)-3,8-diazabicyclo[4.3.0]non-1(6)-ene

1.8g of $N^8$-(ρ-toluenesulfonyl)-3,8-diazabicyclo[4.3.0]non-1(6)-ene, prepared in preparation 8, was dissolved in 30 ml of methanol. 6 ml of 50% aqueous sodium hydroxide solution and 1.5 ml of benzyl bromide were added to the solution and stirred at room temperature for 5 hours. The reaction mixture was concentrated under reduced pressure to remove methanol and then extracted with methylene chloride (30 ml x 3). It was dried ($Na_2SO_4$) and concentrated and then dried under reduced pressure. 2g of the title compound was obtained (yield 85%).
Melting point : 196 ° C
$^1$H-NMR ($CDCl_3$, δ ppm) : 7.80 (2H, d, J = 8.2 Hz), 7.44 (2H, d, J = 8.2 Hz), 7.28 (5H, br. s), 4.01 (4H, s), 3.56 (2H, s), 3.40(2H, s), 2.90(2H, d, J = 5.8Hz), 2.22(2H, t, J = 5.8 Hz).

Preparation 10 : Preparation of $N^3$-(benzyl)-3,8-diazabicyclo[4.3.0]non-1(6)-ene hydrobromide

2g of $N^3$-(benzyl)-$N^8$-($p$-toluenesulfonyl)-3,8-diazabicyclo[4.3.0] pared in Preparation 9, was suspended in 15 ml of 48% hydrobromic acid and 1.5g of phenol, and the reaction mixture was refluxed for 3 hours. After cooling the reaction mixture, 20 ml of water was added the mixture was washed with chloroform (50 ml x 3). The aqueous phase was taken and decolorized by active carbon. The aqueous phase was concentrated under reduced pressure and thus resulting solid was washed with 1 : 1 methanol-ethylether solvent. 1.5g of the title compound was obtained (yield 98%).
Melting point : 205° C(dec.)
$^1$H-NMR ($CDCl_3$, $\delta$ ppm) : 7.29 (5H, br. s), 4.00 (4H, s), 3.55 (2H, s), 3.38(2H, s), 2.91(2H, d, J = 5.8Hz), 2.24(2H, t, J = 5.8 Hz).

Preparation 11 : Preparation of 3,8-diazabicyclo[4.3.0]non-1(6)-ene . dihydrobromide

1.5g of $N^8$-($p$-toluenesulfonyl)-3,8-diazabicyclo[4.3.0]non-1(6)-ene, prepared in Preparation 8, was suspended in 15 ml of 48% hydrobromic acid and 2g of phenol, and the reaction mixture was refluxed for 4 hours. After cooling the reaction mixture, 20 ml of water was added. The mixture was washed with chloroform (40 ml x 3). The aqueous phase was taken and decolorized by active carbon. The aqueous phase was concentrated under reduced pressure and thus resulting solid was washed with 1 : 1 methanol-ethylether solvent. 0.9g of the title compound was obtained (yield 98%).
Melting point : 225~227° C(dec.)

Preparation 12 : Preparation of 3,8-diazabicyclo[4.3.0]non-1(6)-ene dihydrobromide

0.7g of $N^3$-(benzyl)-3,8-diazabicyclo[4.3.0]non-1(6)-ene hydrobromide, prepared in Preparation 10, was dissolved in 20 ml of 5% aqueous acetic acid solution. 0.5g of 10% palladium charcoal in this solution was suspended and the reaction mixture was refluxed under the hydrogen stream for 7 hours. The solid was filtered off. The filtrate was concentrated under reduced pressure and dissolved in 10 ml of 48% bromic acid. By concentrating the solution under reduced pressure again, 0.5g of the title compound was obtained (yield 73%).
Melting point : 225~227° C(dec.)

Preparation 13 : Preparation of 3-methyl-3,7-diazabicyclo[3.3.0]oct-1(5)-ene dihydrobromide

3,7-Diazabicyclo[3.3.0]oct-1(5)-ene dihydrobromide (0.81g) which was prepared in Preparation 2 was dissolved in water (10 ml). To this solution 35% formaline (0.3 ml) and formic acid (10 ml) were added and then refluxed for 4 hours. The solvents were distilled off and the resulting solid was washed with isopropylalcohol (20 ml) and ethyl ether (20 ml) to give the title compound (0.81g, yield 94%).
Melting point : 185~187° C.

By using the diazabicycloamine compounds of the above general formula (III), prepared accoriding to the above preparations, quinolone compounds of the above general formula (I) and their salts were prepared.

Examples

Example 1 : Preparation of (-)-9-fluoro-3(*S*)-methyl-10-[3,7-diazabicyclo[3.3.0]oct-1(5)-en-3-yl]-7-oxo-2,3-dihydro-7*H*-pyrido[1.2.3-*de*]-1,4-benzoxazine-6-carboxylic acid (KR-10759)

0.28g of (-)-9,10-difluoro-3(*S*)-methyl-7-oxo-2,3-dihydro-7*H*-pyrido[1.2.3-*de*]-1,4-benzoxazine-6-carboxylic acid was dissolved in 3 ml of pyridine. 0.5g of 3,7-diazabicyclo[3.3.0]oct-1(5)-ene dihydrobromide was added hereto and then the reaction mixture was refluxed for 10 hours. The reaction mixture was concentrated under reduced pressure to remove pyridine. 2 ml of toluene was added hereto and concentrated under reduced pressure. This step was repeated three times. The residue was washed with 1 : 1 ethanol-ethylether solvent to obtain 0.32g of the title compound (yield 86%).
Melting point : 196° C(dec.)

$^1$H-NMR ($CDCl_3$ + $CD_3COOD$, $\delta$ ppm) : 8.85 (1H, s), 7.69 (1H, dd, J = 14.2, 1.8 Hz), 4.60 (4H, s), 4.51 (1H, m), 4.31 (2H, br. s), 4.21 (4H, s), 1.65 (3H, d, J = 6.6 Hz).

Example 2 : Preparation of(-)-8-amino-9-fluoro-3(*S*)-methyl-10-[3,7-diazabicyclo[3.3.0]oct-1(5)-en-3-yl]-7-oxo-2,3-dihydro-7*H*-pyrido[1.2.3-*de*]-1,4-benzoxazine-6-carboxylic acid

0.3g of (-)-(8-amino-9,10-difluoro-3(*S*)-methyl-7-oxo-2,3-dihydro-7*H*-pyrido [1.2.3-*de*]-1,4-benzoxazine-6-carboxylic acid was dissolved in 3.5 ml of pyridine, and 0.6g of 3,7-diazabicyclo[3.3.0]oct-1(5)-ene dihydrobromide was added hereto. The reaction mixture was refluxed for 5 hours and concentrated under reduced pressure to remove pyridine. 3 ml of toluene was added hereto and then it was concentrated under reduced pressure. This step was repeated three times. The residue was washed with 1 : 1 ethanol-ethylether solvent to obtain 0.31g of the title compound (yield 79%).
Melting point : 214°C(dec.)
$^1$H-NMR ($CDCl_3$ + $CD_3COOD$, $\delta$ ppm) : 8.02 (1H, s), 4.57 (4H, s), 4.50 (1H, m), 4.20 (4H, s), 1.65 (3H, d, J = 6.6 Hz).

Example 3 : Preparation of (-)-9-fluoro-3(*S*)-methyl-10-[7-methyl-3,7-diazabicyclo[3.3.0]oct-1(5)-en-3-yl]-7-oxo-2,3-dihydro-7*H*-pyrrido[1.2.3-*de*]-1,4-benzoxazine-6-carboxylic acid hydrochloride (KR-10800)

0.1g of (-)-9-fluoro-3(*S*)-methyl-10[3,7-diazabicyclo[3.3.0]oct-1(5)-en-3-yl]-7-oxo-2,3-dihydro-7H-pyrido-[1.2.3-*de*]-1,4-benzoxazine-6-carboxylic acid was dissolved in the mixture of 3 ml of 36% aqueous formaline solution and 3 ml of formic acid, and then the reaction mixture was refluxed for 2 hours. After removing the solvnent under reduced pressure, 10 ml of isopropanol and 3 ml of hydrochloric acid were added hereto, and it was refluxed for 1 hour. The solvent was removed under reduced pressure and the residue was washed with 1 : 1 ethannol-ethylether solvent to obtain 60 mg of the title compound (yield 58%).
Melting point : 271~275°C(dec.)
$^1$H-NMR ($CDCl_3$ + $CD_3COOD$, $\delta$ ppm) : 8.12 (1H, s), 7.80 (1H, d, J = 14.1 Hz), 4.87 (2H, br. s), 4.72 (4H, s), 4.51 (1H, m), 4.40 (2H, br. s), 4.33 (2H, br. s), 3.22 (3H, s), 1.68 (3H, d, J = 6.6 Hz).

Example 4 : Preparation of (i-)-9-fluoro-3(*S*)-methyl-10-{8-[3,8-diazabicyclo [4.3.0]non-1(6)-en-3-yl]}-7-oxo-2,3-dihydro-7*H*-pyrido[1.2.3-*de*]-1,4-benzoxamine-6-carboxylic acid

0.3g of (--)-9,10-difluoro-3(*S*)-methyl-7-oxo-2,3-dihydro-7*H*-pyrido[1.2.3-*de*]-1,4-benzoxazine-6-carboxylic acid, 0.5g of N3-benzyl-3,8-diazabicyclo[4.3.0]non-1(6)-ene hydrobromide and 0.6g of 1,8-diazabicyclo [5.4.0] undec-7-ene (DBU) were suspended in 25 ml of acetonitrile, and then the reaction mixture was refluxed for 5 hours. The reaction mixture was kept overnight at room temperature, and the produced precipitate was filtered and was washed with 1 : 1 ethanol-ethylether solvent to obtain 0.35g of reaction product. The reaction product was dissolved in 30 ml of 5% acetic acid solution in ethanol. 0.04g of 10% Palladium charcoal was added hereto and refluxed under hydrogen stream for 5 hours. The solid was filtered off tha the filtrate was concentrated to obtain 0.25g of the title compound(yield 60%).
Melting point : 218~220°C(dec.)
$^1$H-NMR ($CDCl_3$ + $CD_3COOD$, $\delta$ ppm) : 8.87 (1H, s), 7.70 (1H, d, J = 14.2 Hz), 4.58 (4H, s), 4.50 (1H, m), 4.35 (2H, br. s), 3.31 (2H, t, J = 5.8 Hz), 2.50 (2H, t, J = 5.8 Hz), 1.68 (3H, d, J = 6.6 Hz).

Example 5 : Preparation of (-)-9-fluoro-3(*S*)-methyl-10-[7-methyl-3,7-diazabicyclo[3.3.0]oct-1(5)-en-3-yl]-7-oxo-2,3-dihydro-7*H*-pyrido[1.2.3-*de*]-1,4-benzoxazine-6-carboxylic acid

(-)-9,10-Difluoro-3(*S*)-methyl-7-oxo-2,3-dihydro-7*H*-pyrido[1.2.3-*de*]-1,4-benzoxazine-6-carboxylic acid (0.562g), 3-methyl-3,7-diazabicyclo[3.3.0]oct-1(5)-ene dihydrobromide (0.68g) and 1,8-diazabicyclo[5.4.0]-undec-7-ene (DBU, 0.55g) were dissolved in acetonitrile (20 ml) and refluxed for 7 hours. The reaction mixture was kept at room temperature overnight. The produced precipitate was collected by filtration, washed with acetonitrile and methanol and dried in vacuo to give the title compound 0.54g (yield 70%).
Melting point : 264~268°C(dec.)
$^1$H-NMR ($CDCl_3$ + $CD_3COOD$, $\delta$ ppm) : 8.22 (1H, s), 7.84 (1H, d, J = 14.1 Hz), 4.85 (2H, br. s), 4.70 (4H,

s), 4.50 (1H, m), 4.40 (2H, br. s), 4.31 (2H, br. s), 3.21 (3H, s), 1.65 (3H, d, J = 6.6 Hz).

Example 6 : Preparation of (-)-9-fluoro-3-(*S*)-methyl-10-[7-methyl-3,7-diazabicyclo[3.3.0]oct-1(5)-en-3-yl]-7-oxo-2,3-dihydro-7*H*pyrido[1.2.3-*de*]-1,4-benzoxazine-6-carboxylic acid hydrochloride (KR-10800)

(-)-9-Fluoro-3(*S*)-methyl-10-[7-methyl-3,7-diazabicyclo[3.3.0]oct-1(5)-en-3-yl]-7-oxo-2,3-dihydro-7*H*-pyrido[1.2.3-*de*]-1,4-benzoxazine-6-carboxylic acid (0.386g) was dissolved in 5% acetic acid in water (12 ml). To this solution, 35% hydrochloric acid (3 ml) and ethanol, (20 ml)-ethyl ether (10 ml) were added and then the produced precipitate was collected by filtration and dried in vacuo to give the title compound (0.338g, yield 80%).

Melting point : 271~275 °C(dec.)

The quinolone compounds, prepared in the Examples were tested for the antibacterial activities by the agar dilution test method, and the results are shown in Table I and Table II.

The quinolone compounds such as KR-10759 and KR-10800 have much better antibacterial activities against the Gram positive bacteria such as Staphylococcus and Streptococcus than the existing quinolone compounds such as ofloxacin and norfloxacin, and have similar or better antibacterial activity than ciprofloxacin.

These compounds have also good antibacterial activity against Pseudomonas Sp. Furthermore, it is proved that the quinolone compounds according to the present invention have superior antibacterial activity to existing quinolone antibacterial agents methicilline resistant Staphylococcus aureus.

Table I.

| The in vitro antibacterial activity | | | | | |
|---|---|---|---|---|---|
| No. | Bacteria | The minimum inhibition concentration(μg/ml) | | | |
| | | KR-10759 | KR-10800 | Ciprofloxacin | Ofloxacin |
| 1 | Streptococcus pyogenes 308 | 0.781 | 0.781 | 3.125 | 6.25 |
| 2 | Streptococcus pyogenes 77 | 0.098 | 0.391 | 0.781 | 1.563 |
| 3 | Streptococcus faecium MD 8b | 0.195 | 0.195 | 0.781 | 1.563 |
| 4 | Staphylococcus aureus SG 511 | 0.049 | 0.049 | 0.195 | 0.391 |
| 5 | Staphylococcus aureus 285 | 0.195 | 0.098 | 0.391 | 0.391 |
| 6 | Staphylococcus aureus 503 | 0.195 | 0.195 | 0.391 | 0.391 |
| 7 | Escherichia coli O 55 | 0.013 | 0.013 | <0.002 | 0.049 |
| 8 | Escherichia coli DC 0 | 0.195 | 0.098 | 0.195 | 0.781 |
| 9 | Escherichia coli DC 2 | 0.025 | 0.049 | 0.049 | 0.391 |
| 10 | Escherichia coli TEM | 0.025 | 0.049 | <0.002 | 0.098 |
| 11 | Escherichia coli 1507E | 0.025 | 0.025 | <0.002 | 0.098 |
| 12 | Pseudomonas aeruginosa 9027 | 0.781 | 1.563 | 0.391 | 3.125 |
| 13 | Pseudomonas aeruginosa 1592E | 0.391 | 0.781 | 0.195 | 1.563 |
| 14 | Pseudomonas aeruginosa 1771 | 0.781 | 0.781 | 0.195 | 1.563 |
| 15 | Pseudomonas aeruginosa 1771M | 0.098 | 0.049 | 0.098 | 0.391 |
| 16 | Salmonella typhimurium | 0.025 | 0.025 | <0.002 | 0.049 |
| 17 | Klebsiella aerogenes 1082E | 0.025 | 0.025 | <0.002 | 0.013 |
| 18 | Klebsiella aerogenes 1522E | 0.049 | 0.025 | <0.002 | 0.098 |
| 19 | Enterobacter cloacae P 99 | 0.195 | 0.049 | 0.004 | 0.195 |
| 20 | Enterobacter cloacae 1321E | 0.013 | 0.013 | <0.002 | 0.025 |

Table II.

| The in vitro activity against the methicilline resistant bacteria | | | | |
|---|---|---|---|---|
| No. | Methicillin resistant strains | The minimum inhibition concentration (µg/ml) | | |
| | | KR-10759 | KR-10800 | Ofloxacin |
| 1 | Staphylococcus aureus 88 E | 0.195 | 0.195 | 0.391 |
| 2 | Staphylococcus aureus 121 E | 0.098 | 0.098 | 0.195 |
| 3 | Staphylococcus aureus 208 E | 0.195 | 0.098 | 0.391 |
| 4 | Staphylococcus aureus 256 E | 0.098 | 0.098 | 0.195 |
| 5 | Staphylococcus aureus 690 E | 0.049 | 0.049 | 0.195 |
| 6 | Staphylococcus aureus 692 E | 0.098 | 0.098 | 0.098 |
| 7 | Staphylococcus aureus 693 E | 0.098 | 0.049 | 0.195 |
| 8 | Staphylococcus aureus 694 E | 0.195 | 0.098 | 0.195 |
| 9 | Staphylococcus aureus 695 E | 0.098 | 0.098 | 0.195 |
| 10 | Staphylococcus aureus 697 E | 0.025 | 0.049 | 0.098 |
| 11 | Staphylococcus aureus 701 E | 0.098 | 0.098 | 0.195 |
| 12 | Staphylococcus aureus 703 E | 0.098 | 0.049 | 0.195 |
| 13 | Staphylococcus aureus 705 E | 0.098 | 0.098 | 0.391 |
| 14 | Staphylococcus aureus 706 E | 0.098 | 0.049 | 0.391 |
| 15 | Staphylococcus aureus 707 E | 0.049 | 0.049 | 0.391 |
| 16 | Staphylococcus aureus 708 E | 0.049 | 0.049 | 0.391 |
| 17 | Staphylococcus aureus 711 E | 0.098 | 0.098 | 0.391 |
| 18 | Staphylococcus aureus 714 E | 0.098 | 0.049 | 0.391 |
| 19 | Staphylococcus aureus 725 E | 0.098 | 0.049 | 0.391 |

## Claims

1. Quinolone compounds of the general formula I

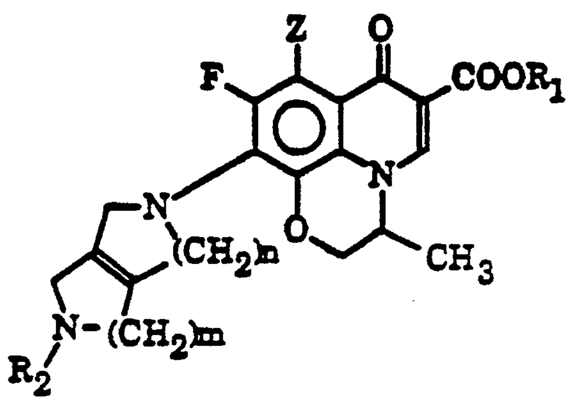

(I)

in which
Z represents halogen or amino,
$R_1$ represents hydrogen or a pharmaceutically acceptable cation,
$R_2$ represents hydrogen, lower alkyl or formyl,
m is an integer of 1 to 3, and
n is 1 or 2,
and in case $R_1$ is hydrogen, pharmaceutically acceptable acid addition salts and the hydrates thereof.

2. A process for the preparation of quinolone compounds of the formula I and the pharmaceutically acceptable salts and hydrates thereof

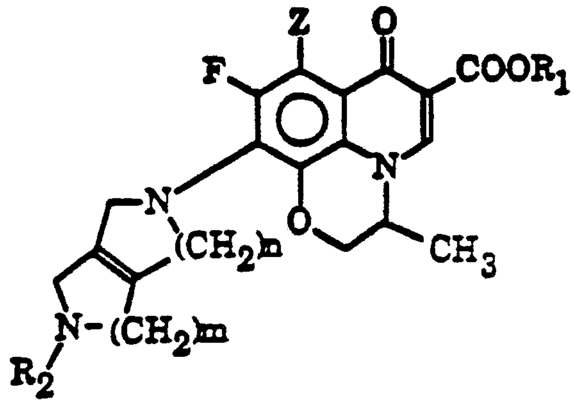

(I)

in which
Z represents halogen or amino
$R_1$ represents hydrogen or a pharmaceutically acceptable cation,
$R_2$ represents hydrogen, lower alkyl or formyl,
m is an integer of 1 to 3, and
n is 1 or 2,
which comprises
($a_1$) condensing a compound of the general formula II

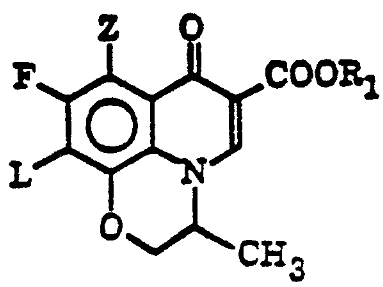

(II)

wherein Z and $R_1$ are as defined above and L represents a leaving group,
with a diazabicycloamine of the general formula III

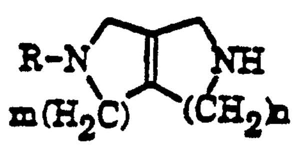

(III)

or its acid salt,
wherein m and n are as defined above and R represents hydrogen, lower alkyl or a protecting group, or
($a_2$) condensing a compound of the formula IIa

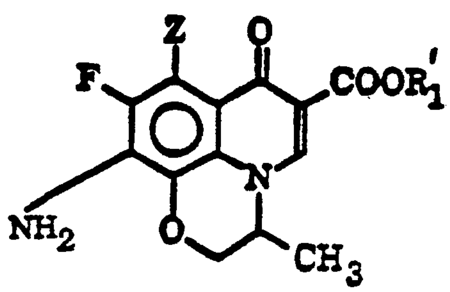

(IIa)

wherein Z has the meaning given above and $R'_1$ stands for a protecting group,

with a compound of the formula IIIa

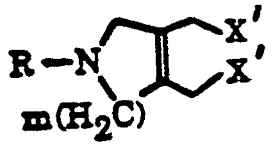

(IIIa)

wherein R and m are as defined above and X′ is a leaving group, whereby a compound of the general formula IV

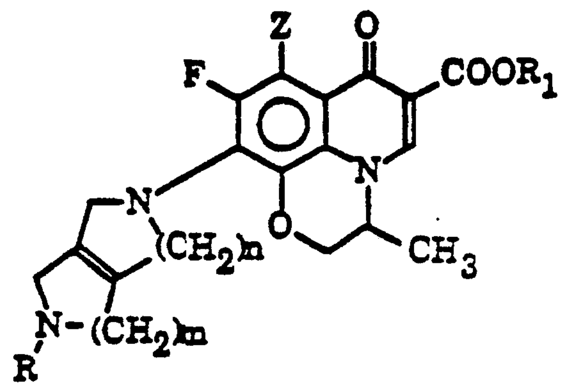

(IV)

is obtained,
in which Z, R, $R_1$, m and n are as defined above, but n is 1 for step $a_2$,
(b) removing the protecting groups and
(c) optionally replacing R = hydrogen by lower alkyl or formyl,
and converting - if desired - the compounds into their pharmaceutically acceptable salts.

3. A pharmaceutical composition active agsint bacterial infections, which contains as effective amount of a quinolone of the general formula I or a pharmaceutically acceptable salt or hydrate thereof.

4. A process for the preparation of a pharmaceutical composition active against bacterial infections, which comprises bringing a quinolone of the formula I or a pharmaceutically acceptable salt or hydrate thereof into a pharmaceutically acceptable administration form with pharmaceuticalla customary excipients or diluents.

5. Use of quinolones of the general formula I or a pharmaceutically acceptable salt or hydrate thereof for the manufacture of a pharmaceutical composition for the treatment of bacterial infections.

6. Method for treating bacterial infections of warm-blooded animals which comprises administering a quinolone of the general formula I or a pharmaceutically acceptable salt or hydrate thereof or a pharmaceutical composition containing such compound.

Claims for the following Contracting States: ES, GR

1. A process for the preparation of quinolone compounds of the formula I and the pharmaceutically acceptable salts and hydrates thereof

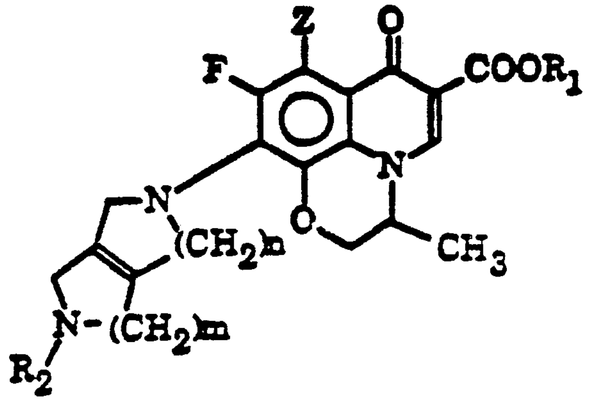

(I)

in which
Z represents halogen or amino
$R_1$ represents hydrogen or a pharmaceutically acceptable cation,
$R_2$ represents hydrogen, lower alkyl or formyl,
m is an integer of 1 to 3, and
n is 1 or 2,
which comprises
($a_1$) condensing a compound of the general formula II

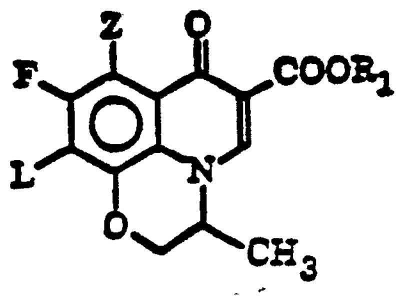

(II)

wherein Z and $R_1$ are as defined above and L represents a leaving group, with a diazabicycloamine of the general formula III

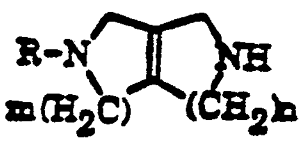

(III)

or its acid salt, wherein m and n are as defined above and R represents hydrogen, lower alkyl or a protecting group, or
($a_2$) condensing a compound of the formula IIa

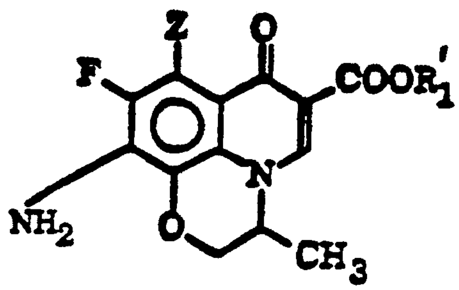

(IIa)

wherein Z has the meaning given above and $R'_1$ stands for a protecting group, with a compound of the formula IIIa

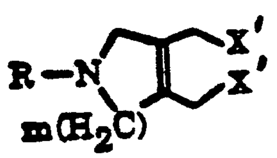

(IIIa)

wherein R and m are as defined above and X' is a leaving group, whereby a compound of the general formula IV

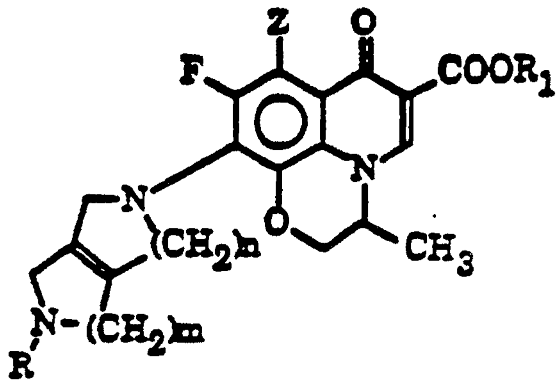

(IV)

is obtained,

in which Z, R, $R_1$, m and n are as defined above, but n is 1 for step $a_2$,

(b) removing the protecting groups and

(c) optionally replacing R = hydrogen by lower alkyl or formyl,

and converting - if desired - the compounds into their pharmaceutically acceptable salts.

2. A process for the preparation of a pharmaceutical composition active against bacterial infections, which comprises bringing a quinolone of the formula I or a pharmaceutically acceptable salt or hydrate thereof into a pharmaceutically acceptable administration form with pharmaceuticalla customary excipients or diluents.

3. Use of quinolones of the general formula I or a pharmaceutically acceptable salt or hydrate thereof for the manufacture of a pharmaceutical composition for the treatment of bacterial infections.

4. Method for treating bacterial infections of warm-blooded animals which comprises administering a quinolone of the general formula I or a pharmaceutically acceptable salt or hydrate thereof or a pharmaceutical composition containing such compound.

European Patent Office

# PARTIAL EUROPEAN SEARCH REPORT

which under Rule 45 of the European Patent Convention shall be considered, for the purposes of subsequent proceedings, as the European search report

Application number: EP 90 12 0241

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 106 489 (WARNER-LAMBERT)<br>* Claim 1; page 26, lines 8-12; page 53, line 26 - page 55, line 3 *<br>-- | 1,3 | C 07 D 519/00<br>A 61 K 31/55<br>A 61 K 31/535//<br>(C 07 D 519/00<br>C 07 D 498:00<br>C 07 D 471:00)<br>(C 07 D 519/00<br>C 07 D 498:00<br>C 07 D 487:00) |
| A | EP-A-0 321 191 (PFIZER)<br>* Claims 1,8 *<br>-- | 1,3 | |
| A | JOURNAL OF HETEROCYCLIC CHEMISTRY, vol. 20, no. 2, March-April 1983, pages 321-329, Heterocorporation, Utah, US;<br>C.J. OHNMACHT, Jr. et al.: "Synthesis and carbon-13 NMR study of 2-benzyl, 2-methyl, 2-aryloctahydropyrrolo [3,4-c] pyrroles and the 1,2,3,5-tetrahydropyrrolo [3,4-c] pyrrole ring system"<br>./. | | TECHNICAL FIELDS SEARCHED (Int. Cl.4)<br>C 07 D 519/00<br>A 61 K 31/00 |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-5

Claims searched incompletely:

Claims not searched: 6

Reason for the limitation of the search:

Method for treatment of the human or animal body by surgery or therapy (See art. 52(4) of the European Patent Convention)

| Place of search | Date of completion of the search | Examiner |
|---|---|---|
| THE HAGUE | 27-01-1991 | VOYIAZOGLOU |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1 03.82

European Patent Office

# PARTIAL EUROPEAN SEARCH REPORT

Application number

EP 90 12 0241



| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| | * Page 325, table IV, compounds 9b, 9c,9d * | 1,3 | |
| | -- | | |
| P,A | EP-A-0 343 560 (WAKUNAGA SEIYAKU KABUSHIKI) | | |
| | * Claim 1; page 5, lines 24-28 * | 1,3 | |
| | ---- | | TECHNICAL FIELDS SEARCHED (Int Cl 4) |